(19) **Europäisches Patentamt / European Patent Office / Office européen des brevets**

(11) **EP 3 220 831 B1**

(12) # EUROPEAN PATENT SPECIFICATION

(45) Date of publication and mention
of the grant of the patent:
**26.06.2019 Bulletin 2019/26**

(21) Application number: **15804594.8**

(22) Date of filing: **17.11.2015**

(51) Int Cl.:
*A61B 8/08* (2006.01)          *A61B 8/00* (2006.01)
*A61N 5/10* (2006.01)          *A61N 7/02* (2006.01)
*G06T 7/00* (2017.01)

(86) International application number:
**PCT/IB2015/058857**

(87) International publication number:
**WO 2016/079659 (26.05.2016 Gazette 2016/21)**

(54) **VISUALIZATION APPARATUS FOR PROPERTY CHANGE OF A TISSUE**

VISUALISIERUNGSVORRICHTUNG ZUR ÄNDERUNG VON GEWEBEEIGENSCHAFTEN

APPAREIL DE VISUALISATION POUR UN CHANGEMENT DE PROPRIÉTÉ DE TISSU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2014 US 201462081361 P**

(43) Date of publication of application:
**27.09.2017 Bulletin 2017/39**

(73) Proprietor: **Koninklijke Philips N.V.**
**5656 AE Eindhoven (NL)**

(72) Inventors:
• **SHAN, Caifeng**
**NL-5656 AE Eindhoven (NL)**
• **HARKS, Godefridus Antonius**
**NL-5656 AE Eindhoven (NL)**
• **BELT, Harm Jan Willem**
**NL-5656 AE Eindhoven (NL)**

• **DELADI, Szabolcs**
**NL-5656 AE Eindhoven (NL)**
• **MCGEE, David L**
**NL-5656 AE Eindhoven (NL)**
• **RANKIN, Darrell L.**
**NL-5656 AE Eindhoven (NL)**

(74) Representative: **Steffen, Thomas**
**Philips Intellectual Property & Standards**
**High Tech Campus 5**
**5656 AE Eindhoven (NL)**

(56) References cited:
| | |
|---|---|
| WO-A1-95/07657 | WO-A1-2010/140069 |
| WO-A1-2013/014583 | WO-A1-2014/060870 |
| US-A- 5 984 881 | US-A1- 2012 004 547 |
| US-A1- 2012 265 069 | US-A1- 2014 081 262 |

**Description**

FIELD OF THE INVENTION

The invention relates to a visualization apparatus for visualizing the property change of a tissue resulting upon application of energy to the tissue. The invention relates further to a system comprising energy source and energy application device for applying energy to a tissue, an ultrasound measurement arrangement and a visualization apparatus.

BACKGROUND OF THE INVENTION

US 2009/0105588 A1 discloses an apparatus, method and system for monitoring and controlling radiotherapy. The radiative source emits energy into a tissue which is absorbed at a target site, to heat the tissue. An ultrasound transducer transmits ultrasound signal to the tissue and receives the reflected ultrasound signal. A signal processor processes the received ultrasound signals and calculates the tissue composition scan or tissue temperature. Ultrasound image, tissue temperature scan and strain image are rendered to determine and/or modify the therapeutic radiative dose based on tissue temperature scan or tissue composition scan. Colorbars indicating the range of temperature change and strain differences in the images, are supporting the interpretation of the displayed images. Document US 2012/0004547 A1 may be considered to disclose the preamble of claim 1.

SUMMARY OF THE INVENTION

It is an object of the present invention to provide an apparatus with improved presentation of a property change of a tissue upon energy application, such that the information is readily absorbable by a person who applies energy to the tissue.

According to the invention this object is achieved by a visualization apparatus for rendering a changing of a property of a tissue under influence of energy applied to the tissue, the apparatus comprising a signal processor for processing measurement signals from ultrasound measurement indicative of the property of the tissue at different locations within the tissue so as to derive a location of an interface between a region with tissue with changed property and a region with tissue with unchanged property, and a rendering device for rendering a representation indicating the location of the interface, wherein two extremities of the representation are indicative of two boundaries defining a thickness of the tissue and wherein a position between the two extremities indicates the location of the interface.

Ultrasound measurements performed on a heart, being that from inside or from outside of the heart, comprise information on anatomical entities like myocardium, blood, epicardial fat, lungs, pericardial liquid, and esophagus, depending on the location imaged by the ultrasound transducer. During energy application to the heart tissue with an energy application device the most important information is the change of the property of the myocardial tissue. As a result of the visualization apparatus comprising a rendering device arranged to render a representation wherein two extremities of the representation are indicative of the two boundaries defining the tissue thickness, the person applying energy to the heart tissue can easily focus on the anatomical entity of interest, which is the myocardium.

In an embodiment of the visualization apparatus the rendering device is further arranged to render the region with the changed property of the tissue upon energy application with a different visual aspect than the region with the unchanged property. The different visual aspects of the two regions may be a difference in color or texture, which makes the information readily absorbable by a person who applies energy to the tissue.

In an embodiment of the visualization apparatus the rendering with different visual aspect of the region with changed property with respect to the region with unchanged property is based on processing of ultrasound measurement signals by the processor. The property of the heart tissue is a physical quantity selected from a group consisting of velocity, velocity gradient and strain rate. These physical quantities are reflecting a change in property of the myocardial tissue upon energy application, resulting in changes of tissue contractility, elasticity and perfusion.

Deriving of the location of the interface between the region with tissue with changed property and the region with tissue with unchanged property can be based on a mean, median, minimum, maximum, absolute or standard deviation of the property. These statistical parameters are representative of the respective physical quantities.

The rendering with different visual aspects of the region with changed property and the region with unchanged property is associated in the preferred embodiment to a difference in the range of 10% to 30% of the physical quantity of the two regions in the tissue. The difference in physical quantity in the given range is representing a significant change of the property of myocardial tissue upon energy application, resulting in tissue denaturation, diminution of tissue contractility, elasticity and perfusion. However, for other types of tissue the difference in physical quantities of the region with changed property with respect to the region with unchanged property may differ. The user of the visualization apparatus may select such range.

In an embodiment of the visualization apparatus the rendering device is arranged to render the two extremities representing the tissue boundaries as two opposite sides of a rectangle. The advantage of the embodiment is that the distinct visual aspect is starting to develop from one side of the rectangle upon energy application to the tissue, and it is advancing towards the opposite side of the rectangle in case the energy application is occurring at an appropriate intensity for a sufficient duration.

Alternative embodiments of the visualization apparatus comprise a rendering device arranged to render the two extremities representing the tissue boundaries as coinciding, wherein the distance between the two boundaries defining the tissue thickness is rendered as a circle, a ring or an ellipse. In such embodiment the distinct visual aspect representing the region with changed property of the tissue upon energy application is starting to develop at a certain location in the geometrical form, and upon covering the geometrical from, the distinct visual aspect returns in the location coincident with where it started, provided that the energy application is occurring at an appropriate intensity for a sufficient duration.

In a further aspect of the invention a system comprises an energy source connected to an energy application device for applying energy to a tissue, an ultrasound measurement arrangement and the visualization apparatus. The main advantage of such a system is that the ultrasound probe of the ultrasound measurement arrangement comprising one or multiple ultrasound transducers can be embedded into the energy application device. This allows localized ultrasound measurement exactly on the site where the energy application occurs on the tissue. Due to the integration of the ultrasound transducers in the energy application device, there is no need for alignment of the ultrasound probe and the energy application device in order to avoid shadowing caused by the energy application device in the ultrasound measurement, and the system allows discerning the region with changed property from the region with unchanged property in the tissue with high accuracy and high fidelity.

The energy application device is arranged to apply the energy to the tissue by one of the modalities selected from ultrasound waves, radiofrequency current, radiofrequency waves, microwaves, or a laser beam. The energy application device is connected to the energy source, which is providing energy in the form of electrical current or electromagnetic radiation. In the energy application device the electrical current can be transformed in ultrasound waves, or in electromagnetic waves in the form of radiofrequency waves, microwaves or light.

In a preferred embodiment, the system is arranged to discontinue energy transmission from the energy source to the energy application device in response to a detection that the second visual aspect corresponding to the region with changed property within the tissue covers the entire interval between the two extremities of the rendered representation. The discontinuation of the energy application is concerning the improvement of the efficiency by avoiding application of energy for an excessive duration. Energy application by ultrasound (e.g. high intensity focused ultrasound), or conditions created by cooling locally the surface of the energy application device when other energy application modalities are used, may create circumstances for initiation of property change within the tissue not originating at the site where the tissue boundary is in contact with the energy application device. Similarly to the conventional energy application without local cooling of the energy application device, where the property change is originating at the site of contact between the energy application device and the tissue, the discontinuation of energy application to the tissue occurs in response to a detection that the second visual aspect corresponding to the region with changed property covers the entire interval between the two extremities of the rendered representation.

In an alternative embodiment, the system may be arranged to reduce the amount of energy transmitted from the energy source to the energy application device in response to a detection that the second visual aspect corresponding to the region of changed property within the tissue approaches the lower extremity of the rendered representation.

In a further embodiment, the system comprises a program controlled processor and a computer program for the processor for enabling a selection of the two boundaries of the tissue representing the two extremities of the rendered representation from an ultrasound image, a tissue velocity image or a strain rate image. Depending on the location where energy application to the tissue is chosen, the anatomy of the heart in combination with the design of the energy application device may give rise to circumstances where multiple myocardial layers are present in the ultrasound measurement. Such examples are energy application in a trabeculated region of the heart, pressing interatrial septum against atria or interventricular septum against ventricle, accessing various locations of the atrial appendage hanging over the ventricle. This embodiment is also relevant for selection of only the myocardial layer which is of interest for energy application, in case that the heart tissue comprises epicardial fat beyond the myocardial layer.

This and other aspects of the invention will be apparent from and elucidated with reference to the embodiments described hereinafter.

BRIEF DESCRIPTION OF THE DRAWINGS

In the drawings:

Fig. 1 shows schematically and exemplarily an embodiment of a visualization apparatus,
Fig. 2a, 2b, 2c, 2d, 2e and 2f show schematically and exemplarily M-mode ultrasound pulse-echo images, tissue velocity and tissue strain rate images,

Fig. 3a, 3b and 3c show schematically and exemplarily sequences of images rendered by embodiments of a visualization apparatus not being an embodiment of the invention,

Fig. 4a and 4b show schematically and exemplarily images rendered by other embodiments of a visualization apparatus according to the invention,

Fig. 5 shows schematically and exemplarily a method for selection of the two boundaries of the tissue from a tissue strain rate image, which are constituting the two opposite sides of the rectangular representation of the tissue,

Fig. 6 shows schematically and exemplarily a measurement system comprising an ultrasound measurement arrangement and a visualization apparatus according to the invention,

Fig. 7 shows schematically and exemplarily a system comprising an energy source connected to an energy application device, an ultrasound measurement arrangement and a visualization apparatus according to the invention,

Fig. 8a shows a schematic diagram for discontinuation of energy transmission from the energy source to the energy application device,

Fig. 8b shows a schematic diagram for reduction of the amount of energy transmitted form the energy source to the energy application device,

Fig. 9a, 9b and 9c show schematically and exemplarily sequences for the detection of the presence of only the second visual aspect in the representation, and

Fig. 10 shows schematically and exemplarily an embodiment of an image rendered for energy application to the tissue with an actively cooled distal tip.

DETAILED DESCRIPTION OF EMBODIMENTS

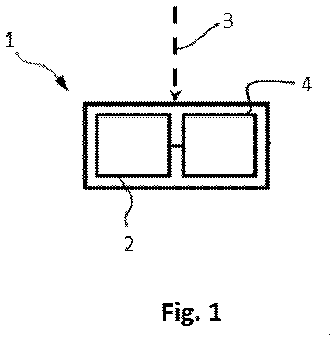

Fig. 1 shows schematically and exemplarily an embodiment of a visualization apparatus 1 comprising a signal processor 2 for processing signals 3 from an ultrasound measurement and a rendering device 4 coupled to the processor for rendering a representation of a property change of a tissue upon energy application. The signal 3 is acquired by an external ultrasound measurement system and it is transmitted to the signal processor 2 of the visualization apparatus 1. The ultrasound measurement signal 3 is preferably derived by means of a real-time motion-mode (M-mode) pulse-echo measurement on a tissue. Alternatively, the M-mode ultrasound information can be extracted from a brightness-mode (B-mode) or a three-dimensional (3D) ultrasound measurement performed by an external ultrasound measurement apparatus. The signal processor 2 is processing the ultrasound measurement signals 3 in a predetermined manner such that the result of the processing is a physical quantity. The physical quantity can be a velocity, a velocity gradient, or a strain rate.

The processor 2 is arranged to process real-time ultrasound data at a rate sufficiently high to avoid aliasing effect in the computation of tissue velocity. From an M-mode ultrasound measurement on the tissue the local phase shift $\Phi$ between two consecutive radiofrequency lines is computed. Assuming that no aliasing occurs, the phase shift is transformed in velocity v of the tissue, according to

$$v = \frac{\Phi}{\pi} \cdot \frac{c \cdot f_p}{4 \cdot f_c} \qquad \text{(Eq. 1)}$$

with c the speed of sound in the tissue, $f_p$ the pulse repetition rate of the ultrasound measurement and $f_c$ the center frequency of the ultrasound transducer. Aliasing is prevented by increasing the pulse repetition rate of the ultrasound measurement to a sufficiently high rate, preferably above 4 kHz.

In another embodiment the strain rate $\varepsilon_r$ within the tissue is computed according to

$$\varepsilon_r = \Delta\Phi \cdot \frac{1}{\pi} \cdot \frac{f_p \cdot f_s}{2 f_c} \qquad \text{(Eq. 2)}$$

with $f_s$ the data sample rate, $\Delta\Phi = \Phi_n - \Phi_{n-1}$ and $n$ the discrete time index.

The rendering device 4 comprises a rendering processor, a memory unit and a display unit. The rendering processor is arranging the data received from the processor 2 for being rendered on the display unit.

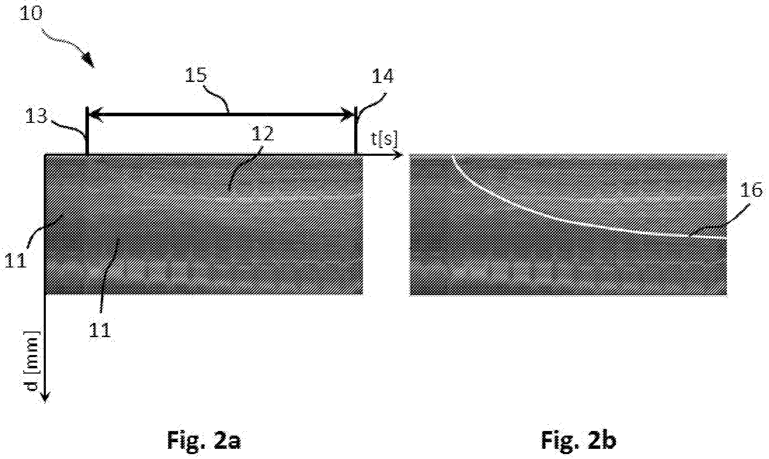

Fig. 2a and 2b show an M-mode ultrasound image 10 of a heart tissue during energy application. The coordinates of an M-mode ultrasound image represent the depth of the measured tissue structure along the vertical axis d, and the temporal change of echogenicity of the same tissue structure along the horizontal axis t. The brightness of the image represents the amplitude of scattered and reflected signal from the imaged structures received by the ultrasound measurement arrangement. In an initial phase, the heart tissue has an intrinsic property 11 characterized by physical quantities.

At the inception of energy application 13 to the heart tissue, the property of the tissue changes 12 in the vicinity of the contact between the energy application device and the heart tissue. The source of the energy for the exemplary description is radiofrequency current. Change of property of the tissue propagates in the depth of the tissue according to a line 16 in Fig. 2b when the intensity of the applied energy is appropriate and the duration of energy application 15 is sufficiently long. The change of tissue property stops propagating in the depth when the energy application is eventually terminated, indicated by reference sign 14. The echogenicity change at the interface represented by line 16 between tissue with changed property during energy application 15 with respect to tissue with unchanged property 11 can be subtle. This makes discrimination of tissue with changed property upon energy application from tissue with unchanged property practically impossible by only using visual interpretation of M-mode ultrasound images.

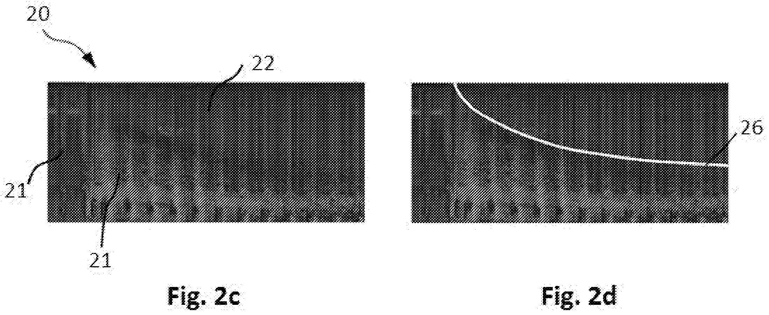

Fig. 2c and 2d show the tissue velocity image 20 of the heart tissue for the same example as presented in the M-mode ultrasound image in Fig. 2a and 2b. The velocity profile in the tissue is derived from M-mode ultrasound measurement by using Eq. 1. Upon energy application to the tissue, the velocity profile in the tissue changes, and two regions that could virtually be separated by the line 26 in Fig. 2d can be identified, representing the tissue with changed property 22 and the tissue with unchanged property 21.

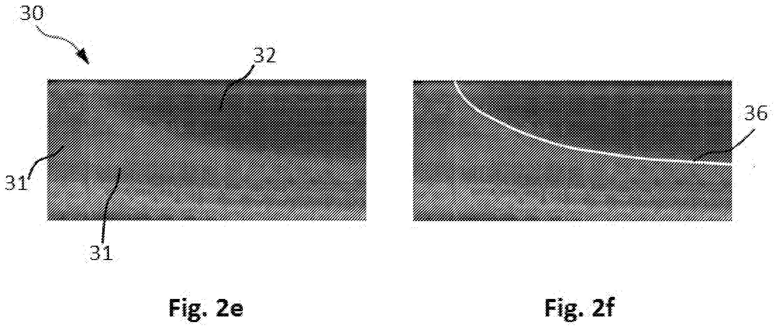

Fig. 2e and 2f show the strain rate image 30 of the heart tissue for the same example as presented in Fig. 2a-2d. The strain rate in the tissue is derived from M-mode ultrasound measurement by using Eq. 2. Upon energy application to the tissue, the strain rate in the tissue changes, and two regions could virtually be separated by the line 36 in Fig. 2f, representing the tissue with changed property 32 and the tissue with unchanged property 31.

Lines 16,26,36 are in most practical cases very difficult or impossible to define based on visual interpretation of the ultrasound 10, the tissue velocity 20 and the strain rate 30 images, due to the large variety of interaction circumstances between the heart tissue and the energy application device. The amount of contact between the heart tissue and the energy application device, hence variation in mechanical restraint of the heart tissue, various contact angles between heart tissue and the energy application device, variation of tissue contractility with anatomical position as well the influence of adjacent organs such as lungs during breathing of living beings, make each and every tissue interaction with the energy application device unique.

It was realized by the inventors that in order to discriminate significant property change upon energy application to a heart tissue, like discrimination between biologically functional and biologically nonfunctional tissue, certain conditions must be fulfilled. At the interface between tissue with changed property 12,22,32 with respect to tissue with unchanged property 11,21,31 there is an abrupt variation of the statistical parameters related to strain rate and tissue velocity. The statistical parameters such as mean, median, minimum, maximum, absolute and standard deviation related to the strain rate and tissue velocity are undergoing an abrupt variation in the range of 10% to 30% at the interface between biologically functional and biologically nonfunctional tissue, whereas the variations within the two regions remain relatively small and without sudden profile change.

The huge dynamic range of strain rate and tissue velocity images due to the large variety of interaction circumstances between the heart tissue and the energy application device makes in most practical cases very difficult to identify this variation of the statistical parameters characterizing the interface between the tissue with changed property upon energy application and tissue with unchanged property.

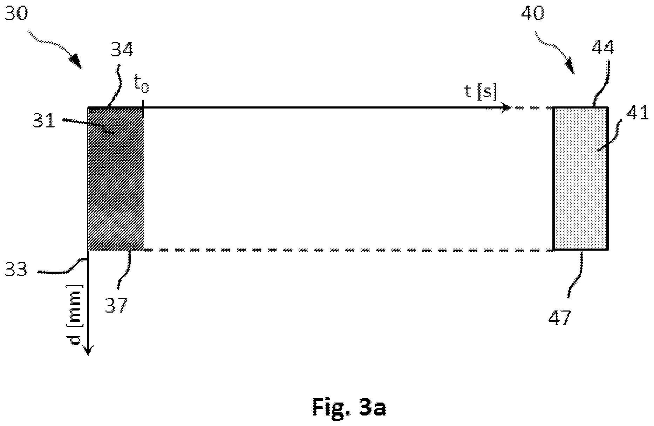

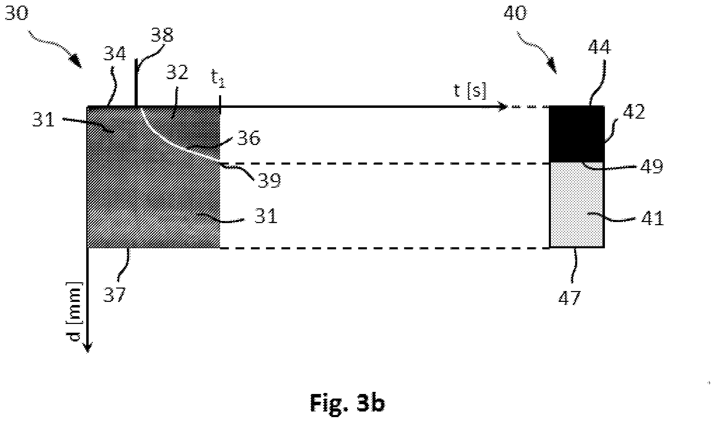

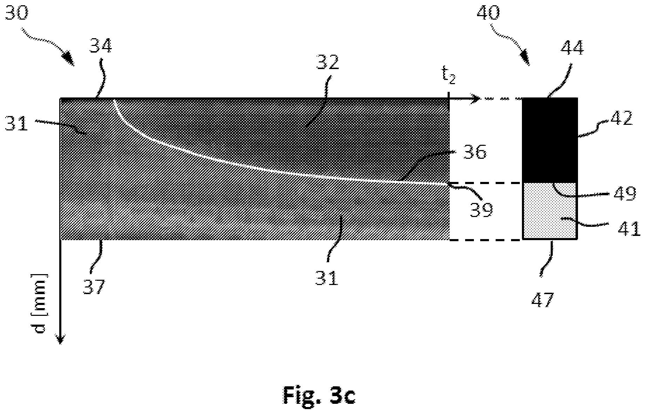

Fig. 3a, 3b, and 3c show a sequence of representations rendered by examples of a visualization apparatus not forming an embodiment of the invention, which overcomes the limitation related to the identification difficulties of the interface between tissue with changed property upon energy application and tissue with unchanged property.

Fig. 3a shows the strain rate image 30 of the heart tissue with unchanged property 31 at time $t_0$, before inception of energy application. Along the vertical axis 33, indicating the depth of the ultrasound measurement, two significant boundaries are defined, the inner wall 34 and the outer wall 37 of the tissue. These two boundaries are defining the two opposite extremities of the rectangular image 40 rendered for the representation of the tissue. The inner wall and the outer wall of the tissue can be identified during the processing of the ultrasound measurement data from signal to noise analysis combined with identification of boundaries between various anatomical entities presenting extremely sudden velocity and strain rate profile changes. The velocity field in tissue computed with Eq. 1 is continuous and homogeneous along the depth of the tissue, unlike the velocity profile of blood. Pericardial liquid shows no ultrasound scattering and no ultrasound reflection, hence the outer wall of the tissue can easily be identified from sharp transition of signal to noise. Also the strain rate computed with Eq. 2 shows particularities at the inner and the outer walls of the heart tissue with respect to blood or pericardial liquid, the variations of the statistical parameters at the two boundaries are very large, potentially reaching a couple orders of magnitude. Between the two opposite sides 44 and 47 of the rectangular representation 40 a first visual aspect 41 is rendered, representing the tissue with unchanged property according to 11,21,31 from the M-mode ultrasound 10, the tissue velocity 20 and the strain rate 30 images, respectively.

Fig. 3b shows the strain rate image 30 of the heart tissue at a moment $t_1$, where $t_1$ is somewhat later than the inception moment 38 of energy application to the tissue. The energy application to the tissue results in modification of strain rate values in close proximity of the energy application device in contact with the inner wall of the heart tissue, while the strain rate values in the region further away from the energy application device and closer to the outer wall of the tissue remain substantially unchanged. The nature of the energy application through radiofrequency current to a tissue is such that first the region in contact with the energy application device undergoes property modification, the energy application reaching consequently deeper regions in time upon energy application at an appropriate intensity for a sufficiently long duration.

At the interface between the tissue with changed property 32 and the tissue with unchanged property 31 there is an abrupt change of the statistical parameters related to strain rate computed with Eq. 2. The statistical parameters such as mean, median, minimum, maximum, absolute and standard deviation are undergoing an abrupt variation in the range of 10% to 30% at that interface 36, whereas the variations within the two regions remain relatively small and without abrupt profile. At the moment $t_1$ the interface between tissue with modified property upon energy application and tissue with unchanged property reaches a certain depth 39. In the rectangular representation 40, a second visual aspect 42 is assigned, corresponding to the region with changed property 32 of the tissue on the strain rate image 30. The region 41 in the rectangular representation 40, corresponding to the region with unchanged property of the tissue 31 on the strain rate image 30, preserves the first visual aspect. The position of the interface 49 between the two different visual aspects 42,41 with respect to the two extremities 44,47 on the rectangular representation 40 is proportional to the position of the interface 39 between the region with changed tissue property 32 and the region with unchanged tissue property 31 with respect to the inner wall 34 and the outer wall 37 of the heart tissue from the strain rate image 30 at any moment of the energy application to the tissue.

Fig. 3c shows the strain rate image 30 of the heart tissue at a moment $t_2$ during energy application to the tissue. The interface 49 between the two different visual aspects 42 and 41, representing the tissue with changed property 32 and the tissue with unchanged property 31 from the strain rate image 30, reached a deeper region of the heart tissue upon application of energy for a longer duration.

In the case of alternative energy application modalities to radiofrequency current such as high intensity focused ultrasound, the modification of the property of the tissue may originate from anywhere between the two extremities 44 and 47 indicative of the two boundaries defining the thickness of the tissue, depending on the characteristics of the energy application device.

Image 40 is preferably rendered stand-alone. However, in alternative embodiments the image 40 may be accompanied by one or a plurality of images from the group consisting of an M-mode ultrasound image 10, a tissue velocity image 20 and a strain rate image 30 of the respective tissue.

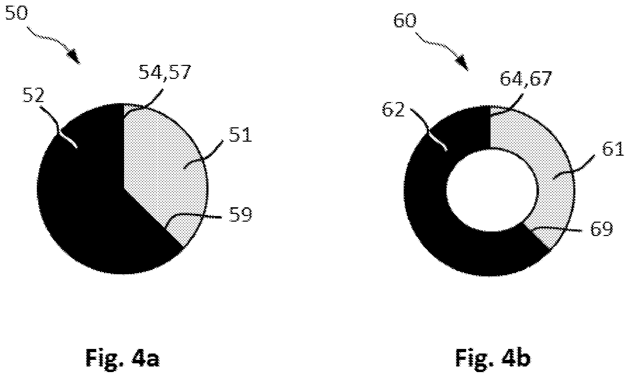

Fig. 4a and 4b show alternative representations rendered by other embodiments of the visualization apparatus according to the present invention, wherein the two boundaries, the inner wall 34 and the outer wall 37 of the tissue from the strain rate image 30 are rendered as coinciding 54,57 and 64,67 in the representations 50 and 60, respectively. The distance between the two boundaries of the tissue 34 and 37, defining the tissue thickness, is rendered as a circle 50 and a ring 60, respectively. The interfaces 59 and 69 between the two different visual aspects 52,51 in the representation 50 and 62,61 in the representation 60 signify the interface 39 between the tissue with changed property 32 upon energy application and the tissue with unchanged property 31 from the strain rate image 30. The lengths of the two segments with different visual aspects 52,51 in the representation 50 and 62,61 in the representation 60 are proportional with the depths of the tissue with changed property and the tissue with unchanged property with respect to the tissue thickness defined by the distance between the inner wall 34 and the outer wall 37 in the strain rate image 30. Other geometrical form such as an ellipse is suitable for similar embodiment.

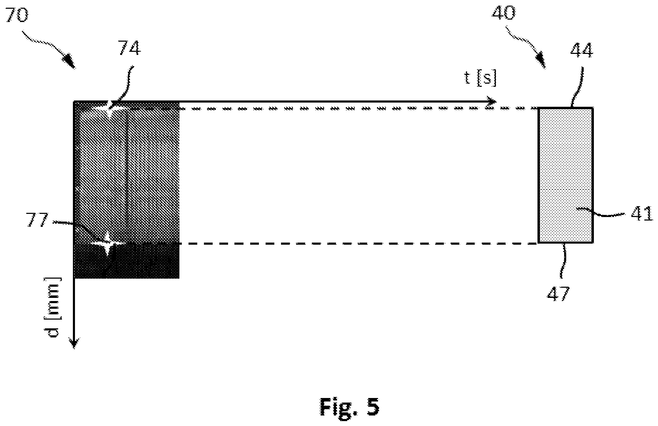

Fig. 5 shows an embodiment for selection of the two boundaries of a heart tissue on the strain rate image 70, the inner wall 74 and the outer wall 77, in case that multiple anatomical entities are positioned in front and beyond the heart tissue in the ultrasound measurement. The different anatomical entities may be structures adjacent to the outer wall of the heart tissue such as lung, esophagus, epicardial fat. The visualization apparatus comprises a program controlled processor and a computer program for the processor enabling selection of the inner wall 74 and outer wall 77 of the tissue from at least one of the following images: an M-mode ultrasound image, a tissue velocity image and a strain rate image. The selected boundaries 74 and 77 constitute the opposite sides 44 and 47 of the rendered rectangular representation 40 of the heart tissue.

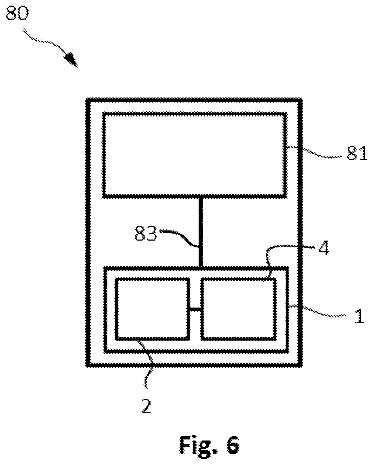

Fig. 6 shows a measurement system 80 comprising an ultrasound measurement arrangement 81 and a visualization apparatus 1. The ultrasound measurement arrangement 81 comprises an ultrasound pulser/receiver unit connected to an ultrasound probe. The ultrasound pulser/receiver unit is arranged to send an electrical pulse to the ultrasound transducer located in the ultrasound probe, which transforms the electrical pulse in ultrasound waves and sends the ultrasound waves into the tissue. The ultrasound waves are scattered and reflected back from the tissue to the ultrasound transducer, which transforms the ultrasound signal to electrical signal and transmits it to the pulser/receiver unit. The pulser/receiver unit provides the ultrasound measurement signals to the signal processor 2 of the visualization apparatus 1 through connection 83. The signal processor 2 is processing the ultrasound measurement signals and the rendering device 4 coupled to the processor is rendering a representation 40 shown schematically in Fig. 3a-3c, for discerning a region of tissue with changed property upon energy application to the tissue from the region with unchanged property, such that the information is readily absorbable by a person who applies energy to the tissue.

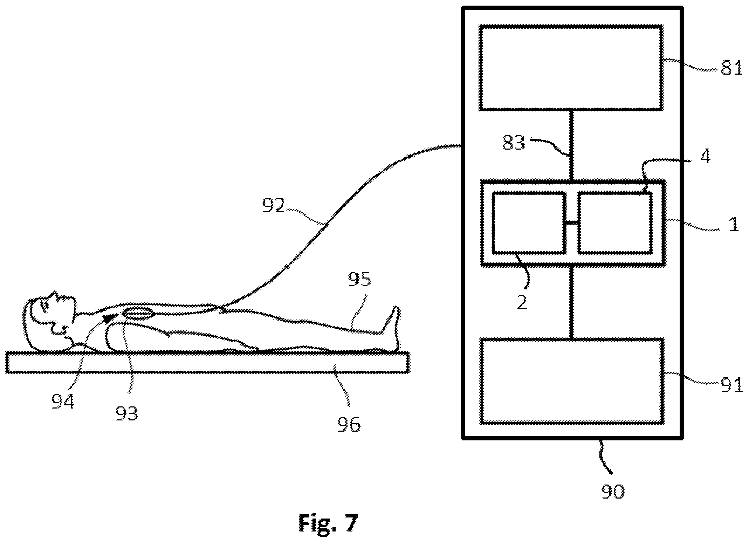

Fig. 7 shows schematically and exemplarily a system 90 comprising an energy source 91 connected to an energy application device 92 for applying energy to a tissue, an ultrasound measurement arrangement 81 and a visualization apparatus 1. The energy source 91 is arranged to provide energy to the energy application device 92 in the form of electrical current or electromagnetic radiation. In the energy application device the electrical current can be transformed in ultrasound waves, radiofrequency waves, microwaves or light. In the case of energy application to the tissue by a laser beam, the energy source can provide the energy to the energy application device directly in electromagnetic radiation through optical fiber, or it can provide electrical current which is transformed in electromagnetic radiation by a laser emitting diode integrated in the energy application device. In the exemplary description, the energy application device 92 is applying radiofrequency current with its distal tip 93 to the heart 94 tissue of a human being 95 laying on the surface of a bed 96.

The ultrasound measurement arrangement 81 comprising an ultrasound pulser/receiver unit connected to one or a plurality of ultrasound transducers integrated into the distal tip 93 of the energy application device 92, provides the ultrasound signal to the visualization apparatus 1 through connection 83.

Due to the integration of the ultrasound transducers into the distal tip 93 of the energy application device 92, the ultrasound measurement is taking place exactly on the location where the energy application occurs to the tissue, hence there is no need for alignment of the ultrasound probe with respect to the energy application device in order to avoid shadowing and/or ringing artifacts caused by the energy application device in the ultrasound measurement. Therefore, discrimination between the region with changed property of the tissue upon energy application from the region with unchanged property is possible with increased accuracy and fidelity.

The ultrasound transducers integrated into the distal tip 93 of the energy application device 92 may be single-piston conventional piezoelectrical transducers, phased array piezoelectrical transducers or capacitive micro-machined ultrasound transducers (CMUT).

In the preferred embodiment the ultrasound measurement is an M-mode pulse-echo measurement such as shown in Fig. 2a, however the M-mode ultrasound measurement signal can be extracted from B-mode or 3D ultrasound measurement modalities. M-mode pulse-echo measurement provides information on the change of position in time of structures within the tissue. This measurement data is an ideal input for the signal processor of the visualization apparatus for processing an output in the form of a physical quantity such as velocity, velocity gradient or strain rate. The change of these physical quantities or related statistical parameters such as mean, median, minimum, maximum, absolute and standard deviation can be indicative of a property change of the tissue upon energy application.

The heart of a living being presents characteristic motion due to intrinsic contraction. This motion results in interaction of the energy application device with the heart tissue when the energy application device is in contact with the inner wall of the heart. The cyclical variation of the interaction is advantageous for the signal processor of the visualization apparatus processing output in physical quantities such as velocity, velocity gradient or strain rate, since the repetitive nature of the interaction presents repetitive patterns in the physical quantities, directly related to the contraction and the relaxation phases of the heart. In case of other tissue types which do not present intrinsic motion, it is preferred that an external cyclical motion is applied on the energy application device with respect to the static tissue.

The ultrasound measurement on the heart tissue can be performed at all time with or without energy application to the tissue. In case of no energy application to the tissue, the rendering device 4 of the visualization apparatus 1 is arranged to render a representation 40 with two extremities 44,47 and a single visual aspect 41 between the two extremities for the tissue with unchanged property, as shown in Fig. 3a. During energy application to the tissue a second visual aspect 42 is rendered in the representation 40, indicating the region with changed property of the tissue upon energy application, as exemplarily shown in Fig. 3b.

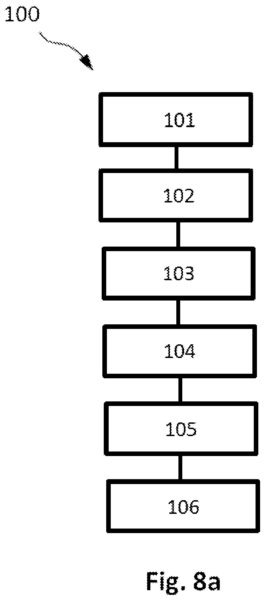

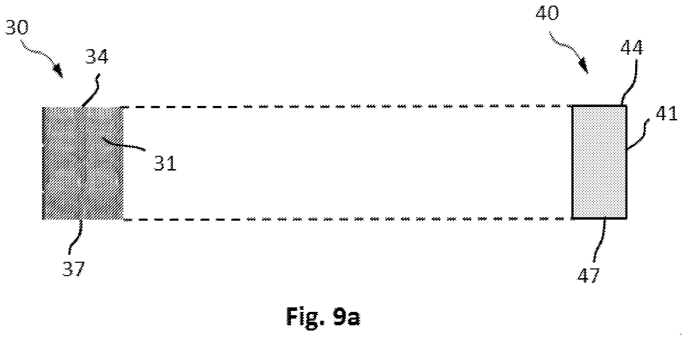

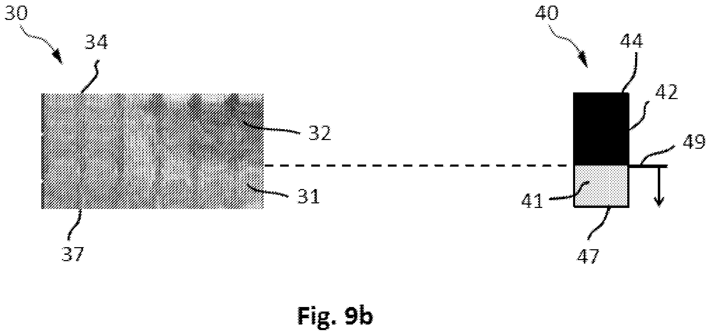

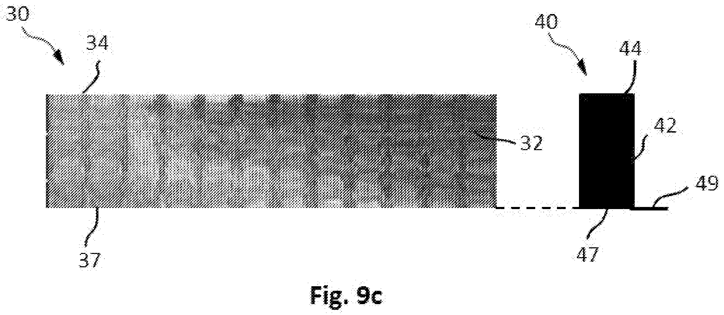

Fig. 8a shows a schematic diagram 100 of the functional use of the system 90. Ultrasound measurement is started in step 101, which can in the first instance assist positioning the distal tip 93 of the energy application device 92 with respect to the heart 94 tissue, after which it provides ultrasound measurement signal from the tissue. In step 102 the signal processor 2 is processing ultrasound measurement signals transferred from the ultrasound measurement arrangement 81 through connection 83. The processor is further computing physical quantities and their related statistical parameters. The two boundaries of the heart tissue, the inner wall 34 and the outer wall 37, are detected and the two extremities 44 and 47 of the rendered representation 40 are assigned accordingly (Fig. 9a). A first visual aspect 41 is rendered between the two extremities 44,47 in the rectangular representation 40, corresponding to the tissue with unchanged property 31. In step 103 the energy application to the heart 94 tissue starts. The distal tip 93 of the energy application device 92, which is connected to the energy source 91 of the system 90, applies energy to the tissue. Upon energy application, there is a potential that the signal processor 2 detects change of the physical quantities and their related statistical parameters in the range of 10% to 30% within the thickness of the heart tissue defined by the inner wall 34 and the outer wall 37. In that case, in step 104 a second visual aspect 42 is assigned to the region with changed property of the tissue in the representation 40 (Fig. 9b), with the interface 49 between the two different visual aspects 42,41 representing proportionally the depth of tissue with changed property and the depth of tissue with unchanged property with respect to the thickness of the heart tissue. Upon energy application at suitable intensity and for a sufficiently long duration, the interface 49 between the two different visual aspects 42,41 is approaching the lower extremity 47 of the representation 40. When the interface 49 reaches the lower extremity 47 (Fig. 9c), the first visual aspect 41 disappears and only the second visual aspect 42 remains present in the representation 40, which indicates that the property of the heart tissue changed throughout the entire thickness of the tissue. At that moment, in step 105 a processor coupled to the rendering device 4 detects that only the second visual aspect 42 is present in the representation 40. In step 106 the processor sends a signal to the energy source 91, which discontinues providing energy to the energy application device 92 upon reception of the signal.

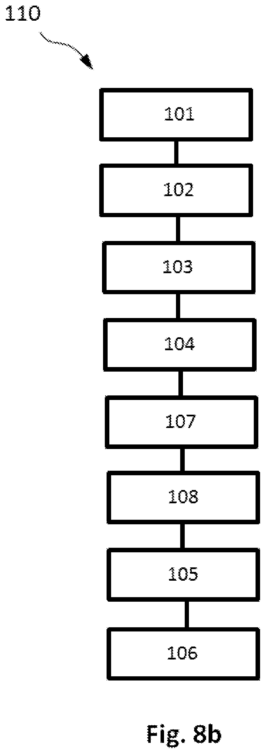

In an alternative functional use of the system 90, shown on the schematic diagram 110 in Fig. 8b, the system 90 may be arranged in step 108 to reduce the amount of energy transmitted from the energy source 91 to the energy application device 92 in response to a detection in step 107 that the second visual aspect 42 corresponding to the region of changed property within the tissue approaches the lower extremity 47 of the rendered representation 40. In the preferred embodiment the reduction of the amount of energy transmitted from the energy source 91 to the energy application device 92 is triggered when the second visual aspect 42 corresponding to the region of changed property within the tissue covers two-thirds of the interval between the two extremities 44,47. The reduction of the amount of energy may be according to a linear function, or according to any other preprogrammed relationship of the position of the interface 49 with respect to the two extremities 44,47 of the rendered representation 40.

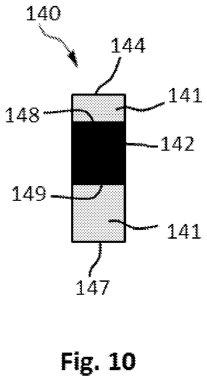

Fig. 10 shows an alternative embodiment of the rendered representation 140 of the present invention for energy application to a tissue with an energy application device which has an actively cooled distal tip. The active cooling may comprise open or closed irrigation with cooling liquid such as saline solution. In those conditions of energy application to the tissue, circumstances may arise when the property change is not initiating at the inner wall of the heart tissue, even when the distal tip of the energy application device is in contact with that location, but it is initiating somewhere between the inner and outer wall of the tissue. In the exemplary representation 140, the tissue with changed property rendered with a second visual aspect 142 is located between the two extremities 144 and 147. The first visual aspect 141, representing the tissue with unchanged property, is divided into two parts by the second visual aspect 142. The two parts of the first visual aspect 141 are adjacent to the two extremities representing the inner and outer walls of the tissue. The two different visual aspects 141,142 have two interfaces 148 and 149. Upon application of energy at an appropriate intensity and for a sufficiently long duration, the interfaces 148,149 may approach and even reach the extremities 144,147. Discontinuation of the energy application may be triggered by a detection of the disappearance of the first visual aspect from the rendered representation.

The energy application device with actively cooled distal tip may apply energy to the tissue in the form of ultrasound waves, radiofrequency current, radiofrequency waves, microwaves, or a laser beam. The nature of the high intensity focused ultrasound waves may provide similar circumstances even without active cooling of the distal tip of the energy application device.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the internet or other wired or wireless telecommunication systems.

Any reference signs in the claims should not be construed as limiting the scope.

## Claims

1.  A visualization apparatus (1) for rendering a changing of a property of a tissue under influence of energy applied to the tissue, the apparatus comprising a signal processor (2) configured for processing measurement signals from ultrasound measurement (3) indicative of the property of the tissue at different locations within the tissue so as to derive a location of an interface (39) between a region with tissue with changed property and a region with tissue with unchanged property, and a rendering device (4) configured for rendering a representation (50,60) indicating the location of the interface (39) in the representation, wherein the rendering device is arranged to render such that:

    two extremities of the representation (54,57) are indicative of two boundaries defining a thickness of the tissue and wherein a position between the two extremities indicates the location of the interface;
    **characterized in that** the rendering device is arranged such that the two extremities (54,57) indicative of the said tissue boundaries coincide and the distance between the two boundaries defining the tissue thickness is rendered as a circle (50), a ring (60) or an ellipse;

the interface in the representation, (59), signifies the interface (39) between tissue with changed property and tissue with unchanged property, and

wherein the rendering device (4) is arranged to render the region with the changed property (52) of the tissue with a different visual aspect than the region with the unchanged property (51), and wherein the property of the tissue is a physical quantity selected from a group consisting of velocity, velocity gradient and strain rate.

2. The visualization apparatus (1) according to claim 1, wherein the deriving of the location of the interface is based on a mean, median, minimum, maximum, absolute or standard deviation of the property.

3. The visualization apparatus (1) defined in claim 2, wherein the rendering with different visual aspects of the region with changed property (52) and the region with unchanged property (51) is associated to a difference in the range of 10% to 30% of the physical quantity of the two regions in the tissue.

4. A measurement system (80) comprising an ultrasound measurement arrangement (81) and the visualization apparatus (1) according to claim 1.

5. A system (90) comprising an energy source (91) connected to an energy application device (92) for applying energy to the tissue and the measurement system (80) according to claim 4.

6. System (90) according to claim 5, comprising a program controlled processor and a computer program for the processor arranged for enabling a selection of the two boundaries of the tissue (74,77) representing the two extremities (54,57) of the rendered representation (50) from an ultrasound image, a tissue velocity image or a strain rate image (70).

7. System (90) according to claim 5, wherein the energy application device is arranged to apply the energy to the tissue by one of the modalities selected from ultrasound waves, radiofrequency current, radio frequency waves, microwaves, or a laser radiation.

8. System (90) according to claim 5, arranged to discontinue energy transmission from the energy source (91) to the energy application device (92) in response to a detection (105) that a first visual aspect (51) corresponding to tissue with unchanged property dissapears and a second visual aspect (52) corresponding to the region of changed property within the tissue covers the entire interval between the two extremities (54,57) of the rendered representation (50).

9. System (90) according to claim 5, arranged to reduce the amount of energy (108) transmitted from the energy source (91) to the energy application device (92) in response to a detection (107) that a first visual aspect (51) corresponding to tissue with unchanged property reduces and a second visual aspect (52) corresponding to the region of changed property within the tissue approaches the lower extremity (57) of the rendered representation (50).

**Patentansprüche**

1. Visualisierungseinrichtung (1) zum Rendern eines Änderns einer Eigenschaft eines Gewebes unter dem Einfluss von an das Gewebe angelegter Energie, wobei die Einrichtung einen Signalprozessor (2), der konfiguriert ist, um Messsignale von einer Ultraschallmessung (3) zu verarbeiten, die auf die Eigenschaft des Gewebes an verschiedenen Orten innerhalb des Gewebes hinweisend sind, um einen Ort einer Schnittstelle (39) zwischen einer Region mit Gewebe mit einer geänderten Eigenschaft und einer Region mit Gewebe mit einer ungeänderten Eigenschaft abzuleiten, und eine Rendervorrichtung (4) umfasst, die konfiguriert ist, um eine Repräsentation (50,60) zu rendern, die auf den Ort der Schnittstelle (39) in der Repräsentation hinweist, wobei die Rendervorrichtung angeordnet ist, um zu rendern, sodass:

zwei Extreme der Repräsentation (54,57) auf zwei Grenzen hinweisend sind, die eine Dicke des Gewebes definieren, und wobei eine Position zwischen den zwei Extremen auf den Ort der Schnittstelle hinweist;

**dadurch gekennzeichnet, dass** die Rendervorrichtung angeordnet ist, sodass die zwei Extreme (54,57), die auf die Gewebegrenzen hinweisend sind, zusammenfallen und die Distanz zwischen den zwei Grenzen, welche die Gewebedicke definieren, als ein Kreis (50), ein Ring (60) oder eine Ellipse gerendert wird;

die Schnittstelle in der Repräsentation, (59), die Schnittstelle (39) zwischen Gewebe mit einer geänderten Eigenschaft und Gewebe mit einer ungeänderten Eigenschaft bezeichnet, und
wobei die Rendervorrichtung (4) angeordnet ist, um die Region mit der geänderten Eigenschaft (52) des Gewebes mit einer unterschiedlichen visuellen Erscheinungsform zu rendern als die Region mit der ungeänderten Eigenschaft (51), und wobei die Eigenschaft des Gewebes eine physikalische Größe ist, ausgewählt aus einer Gruppe, bestehend aus Geschwindigkeit, Geschwindigkeitsgradienten und Dehnungsrate.

2. Visualisierungseinrichtung (1) nach Anspruch 1, wobei das Ableiten des Ortes der Schnittstelle auf einer durchschnittlichen, mittleren, minimalen, maximalen, absoluten oder Standardabweichung der Eigenschaft basiert.

3. Visualisierungseinrichtung (1), definiert in Anspruch 2, wobei das Rendern mit verschiedenen visuellen Erscheinungsformen der Region mit einer geänderten Eigenschaft (52) und der Region mit einer ungeänderten Eigenschaft (51) zusammenhängt mit einer Differenz im Bereich von 10 % bis 30 % der physikalischen Größe der zwei Regionen im Gewebe.

4. Messsystem (80), umfassend eine Ultraschallmessanordnung (81) und die Visualisierungseinrichtung (1) nach Anspruch 1.

5. System (90), umfassend eine Energiequelle (91), die mit einer Energieanlegungsvorrichtung (92) verbunden ist, um Energie an das Gewebe anzulegen, und das Messsystem (80) nach Anspruch 4.

6. System (90) nach Anspruch 5, umfassend einen programmgesteuerten Prozessor und ein Computerprogramm für den Prozessor, angeordnet für ein Ermöglichen einer Auswahl der zwei Grenzen des Gewebes (74,77), welche die beiden Extreme (54,57) der gerenderten Repräsentation (50) von einem Ultraschallbild, einem Gewebegeschwindigkeitsbild oder einem Dehnungsratenbild (70) repräsentieren.

7. System (90) nach Anspruch 5, wobei die Energieanlegungsvorrichtung angeordnet ist, um die Energie durch eine der Modalitäten, ausgewählt aus Ultraschallwellen, Radio frequenzstrom, Radio frequenzwellen, Mikrowellen oder einer Laserstrahlung, an das Gewebe anzulegen.

8. System (90) nach Anspruch 5, angeordnet, um eine Energieübertragung von der Energiequelle (91) an die Energieanlegungsvorrichtung (92) als Antwort auf eine Erfassung (105) zu unterbrechen, dass eine erste visuelle Erscheinungsform (51), die Gewebe mit einer ungeänderten Eigenschaft entspricht, verschwindet und eine zweite visuelle Erscheinungsform (52), die der Region einer geänderten Eigenschaft innerhalb des Gewebes entspricht, das gesamte Intervall zwischen den beiden Extremen (54,57) der gerenderten Repräsentation (50) bedeckt.

9. System (90) nach Anspruch 5, angeordnet, um die von der Energiequelle (91) an die Energieanlegungsvorrichtung (92) übertragene Energiemenge (108) als Antwort auf eine Erfassung (107) zu reduzieren, dass sich eine erste visuelle Erscheinungsform (51), die Gewebe mit einer ungeänderten Eigenschaft entspricht, reduziert und sich eine zweite visuelle Erscheinungsform (52), die der Region einer geänderten Eigenschaft innerhalb des Gewebes entspricht, dem unteren Extrem (57) der gerenderten Repräsentation (50) nähert.

**Revendications**

1. Appareil de visualisation (1) permettant de restituer un changement d'une propriété d'un tissu sous l'influence d'une énergie appliquée au tissu, l'appareil comprenant un processeur de signal (2) configuré pour traiter des signaux de mesure issus d'une mesure ultrasonore (3) indiquant la propriété de le tissu à différents emplacements dans le tissu afin de déduire l'emplacement d'une interface (39) entre une région avec un tissu ayant une propriété modifiée et une région avec un tissu avec une propriété non modifiée, et un dispositif de restitution (4) configuré pour restituer une représentation (50,60) indiquant l'emplacement de l'interface (39) dans la représentation, dans lequel le dispositif de restitution étant conçu pour restituer de telle sorte que :

   deux extrémités de la représentation (54,57) indiquent deux limites définissant une épaisseur du tissu, et dans lequel une position entre les deux extrémités indique l'emplacement de l'interface ;
   **caractérisé en ce que** le dispositif de restitution est agencé de sorte que les deux extrémités (54,57) indiquant lesdites limites de tissu coïncident et que la distance entre les deux limites définissant l'épaisseur de tissu soit restituée sous la forme d'un cercle (50), d'un anneau (60) ou d'une ellipse ;
   l'interface dans la représentation, (59) signifie l'interface (39) entre un tissu à propriété modifiée et un tissu à propriété non modifiée, et

dans lequel le dispositif de restitution (4) est conçu pour restituer la région du tissu ayant la propriété modifiée (52) avec un aspect visuel différent de la région ayant la propriété non modifiée (51), et dans lequel la propriété du tissu est une quantité physique sélectionné dans un groupe comptant la vitesse, le gradient de vitesse et le taux de contrainte.

2. Appareil de visualisation (1) selon la revendication 1, dans lequel la déduction de l'emplacement de l'interface est basée sur un écart moyen, médian, minimum, maximum, absolu ou type de la propriété.

3. Appareil de visualisation (1) selon la revendication 2, dans lequel la restitution avec différents aspects visuels de la région ayant une propriété modifiée (52) et de la région ayant une propriété non modifiée (51) est associé à une différence dans l'intervalle de 10 % à 30 % de la quantité physique des deux régions dans le tissu.

4. Système de mesure (80) comprenant un agencement de mesure à ultrasons (81) et l'appareil de visualisation (1) selon la revendication 1.

5. Système (90) comprenant une source d'énergie (91) connectée à un dispositif d'application d'énergie (92) pour appliquer de l'énergie au tissu et au système de mesure (80) selon la revendication 4.

6. Système (90) selon la revendication 5, comprenant un processeur commandé par programme et un programme informatique pour le processeur conçu pour permettre une sélection des deux limites du tissu (74,77) représentant les deux extrémités (54,57) de la représentation (50) restituée à partir d'une image ultrasonore, d'une image de vitesse de tissu ou d'une image de taux de contrainte (70).

7. Système (90) selon la revendication 5, dans lequel le dispositif d'application d'énergie est conçu pour appliquer l'énergie au tissu par l'une des modalités choisies parmi des ondes ultrasonores, du courant radiofréquence, des ondes radiofréquences, des micro-ondes ou un rayonnement laser.

8. Système (90) selon la revendication 5, agencé pour interrompre la transmission d'énergie de la source d'énergie (91) au dispositif d'application d'énergie (92) en réponse à une détection (105) qu'un premier aspect visuel (51) correspondant à du tissu à propriété non modifiée disparaît, et qu'un second aspect visuel (52) correspondant à la région à propriété modifiée dans le tissu couvre tout l'intervalle entre les deux extrémités (54,57) de la représentation (50) restituée.

9. Système (90) selon la revendication 5, conçu pour réduire la quantité d'énergie (108) transmise de la source d'énergie (91) au dispositif d'application d'énergie (92) en réponse à une détection (107) qu'un premier aspect visuel (51) correspondant à un tissu à propriété non modifiée est réduit, et qu'un second aspect visuel (52) correspondant à la région à propriété modifiée dans le tissu se rapproche de l'extrémité inférieure (57) de la représentation restituée (50).

**Fig. 1**

**Fig. 2a**

**Fig. 2b**

**Fig. 2c**

**Fig. 2d**

Fig. 2e

Fig. 2f

EP 3 220 831 B1

Fig. 3a

**Fig. 3b**

**Fig. 3c**

EP 3 220 831 B1

**Fig. 4a**

**Fig. 4b**

**Fig. 5**

**Fig. 6**

81

4

1

91

90

83

2

92

95

96

93

94

Fig. 7

100

101

102

103

104

105

106

Fig. 8a

110

```
┌─────────────────┐
│       101       │
└─────────────────┘
         │
┌─────────────────┐
│       102       │
└─────────────────┘
         │
┌─────────────────┐
│       103       │
└─────────────────┘
         │
┌─────────────────┐
│       104       │
└─────────────────┘
         │
┌─────────────────┐
│       107       │
└─────────────────┘
         │
┌─────────────────┐
│       108       │
└─────────────────┘
         │
┌─────────────────┐
│       105       │
└─────────────────┘
         │
┌─────────────────┐
│       106       │
└─────────────────┘
```

Fig. 8b

Fig. 9a

**Fig. 9b**

EP 3 220 831 B1

**Fig. 9c**

Fig. 10

**EP 3 220 831 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090105588 A1
- US 20120004547 A1